# EUROPÄISCHE PATENTSCHRIFT

(11) **EP 2 656 801 B1**
(45) Veröffentlichungstag und Bekanntmachung des Hinweises auf die Patenterteilung: **14.05.2014**
(21) Anmeldenummer: 12165086.5
(22) Anmeldetag: 23.04.2012
(51) Int. Cl.: A61B 17/16, A61C 1/14

(54) **Werkzeughaltevorrichtung für ein medizinisches, insbesondere dentales oder chirurgisches, Handstück**
Tool holding device for a medical, in particular dental or surgical handpiece
Dispositif de fixation d'outil pour une pièce à main médicale, notamment dentaire ou chirurgicale

(43) Veröffentlichungstag der Anmeldung: 30.10.2013
(73) Patentinhaber: W & H Dentalwerk Bürmoos GmbH, 5111 Bürmoos (AT)
(72) Erfinder: Jindra, Thomas, 5121 Ostermiething (AT)
(74) Vertreter: Benda, Ralf

(56) Entgegenhaltungen:
- DE-A1-102010 025 826
- DE-C1- 10 130 900
- US-B2- 7 575 432

## Beschreibung

Die vorliegende Erfindung betrifft eine Werkzeughaltevorrichtung zum Halten eines medizinischen, insbesondere dentalen oder chirurgischen, Werkzeugs und eine medizinisches, insbesondere dentales oder chirurgisches, Handstück, vorzugsweise zur Verwendung bei implantologischen Behandlungen, mit einer derartigen Werkzeughaltevorrichtung.

Aus dem Patent US 7,575,432 B2 ist ein medizinisches, insbesondere dentales oder chirurgisches, Handstück, vorzugsweise zur Verwendung bei implantologischen Behandlungen, bekannt, dessen Werkzeughaltevorrichtung im Handstückkopf eine formschlüssige, polygonale Drehmomentübertragung auf das Werkzeug aufweist. Die formschlüssige, polygonale Drehmomentübertragung ermöglich eine vorzügliche Übertragung von, vorzugsweise hohen, Drehmomenten, wie sie bei implantologischen Behandlungen benötigt werden, insbesondere ohne Beschädigung des Werkzeugs.

Die Patentschrift DE 101 30 900 C1 offenbart ein gerades Handstück mit einer durch einen Rotationsantrieb antreibbaren Spannhülse, in der eine Spannzange mit Sperrwalzen aufgenommen ist. Durch eine Relativbewegung zwischen der Spannhülse und der Spannzange mit den Sperrwalzen und dem darin aufgenommenen Werkzeug wird das Werkzeug in dem Handstück fixiert und das Drehmoment von der Spannhülse über die Spenwalzen auf das Werkzeug übertragen.

Der vorliegenden Erfindung liegt die Aufgabe zugrunde, eine Werkzeughaltevorrichtung zum Halten eines medizinischen, insbesondere dentalen oder chirurgischen, Werkzeugs und ein medizinisches, insbesondere dentales, Handstück, vorzugsweise zur Verwendung bei implantologischen Behandlungen, mit einer derartigen Werkzeughaltevorrichtung zu schaffen, die eine formschlüssige, polygonale Drehmomentübertragung auf das Werkzeug aufweist, so dass insbesondere hohe Drehmomente übertragbar sind, wobei zumindest eine Abmessung, insbesondere die Höhe, der Werkzeughaltevorrichtung oder des Handstückkopfs, verringert sein soll, um damit eine verbesserte Handhabung des Handstücks im Mundraum eines Patienten zu ermöglichen.

Diese Aufgabe wird durch eine Werkzeughaltevorrichtung zum Halten eines medizinischen, insbesondere dentalen oder chirurgischen, Werkzeugs gelöst, die umfasst: Eine in eine Drehbewegung versetzbare Werkzeughülse, deren Innenbohrung zur Aufnahme zumindest eines Teils eines ersten, im Querschnitt runden Abschnitts des Werkzeugschafts ausgebildet ist, eine mit der Werkzeughülse verbundene Drehmomentübertragungsvorrichtung, die zur, insbesondere formschlüssigen, Aufnahme eines zweiten, polygonalen Abschnitts des Werkzeugschafts und zur Übertragung eines Drehmoments auf das Werkzeug ausgebildet ist, und einen Klemmrollen-Haltemechanismus, der aufweist: Zumindest eine, in einem Durchbruch der Werkzeughülse aufgenommene Klemmrolle und eine in eine Drehbewegung versetzbare Klemmhülse, welche die zumindest eine Klemmrolle und die Werkzeughülse umgibt, wobei an der Innenwand der Klemmhülse eine im Querschnitt mit sich verändernder Tiefe in die Innenwand gewölbte Vertiefung vorgesehen ist, in welcher die Klemmrolle aufnehmbar ist, und wobei die Klemmhülse einerseits und die zumindest eine Klemmrolle und die Werkzeughülse andererseits derart relativ zueinander drehbar angeordnet sind, dass durch eine Relativbewegung zwischen der Klemmhülse einerseits und der zumindest einen Klemmrolle und der Werkzeughülse andererseits die zumindest eine Klemmrolle von einer ersten Position mit einer ersten Tiefe in der gewölbten Vertiefung der Innenwand der Klemmhülse in eine zweite Position mit einer zweiten Tiefe, die geringer ist als die erste Tiefe, und dadurch in Richtung der Innenbohrung der Werkzeughülse bewegbar ist, so dass ein in der Werkzeughülse aufgenommener Werkzeugschaft durch den Klemmrollen-Haltemechanismus lösbar in der Werkzeughaltevorrichtung befestigbar ist.

Das Vorsehen der polygonalen Drehmomentübertragungsvorrichtung und des Klemmrollen-Haltemechanismus garantiert weiterhin eine vorzügliche Übertragung von, vorzugsweise hohen, Drehmomenten, wie sie bei implantologischen Behandlungen benötigt werden, insbesondere ohne Beschädigung des Werkzeugs. Im Unterschied zu den aus dem Stand der Technik bekannten Werkzeughaltevorrichtungen mit polygonaler Drehmomentübertragungsvorrichtung auf das Werkzeug benötigt die erfindungsgemäße Werkzeughaltevorrichtung aufgrund des Vorsehens des Klemmrollen-Haltemechanismus jedoch keine Löse- oder Entriegelungsvorrichtung, mit deren Hilfe das in der Werkzeughaltevorrichtung eingespannte Werkzeug aus der Werkzeughaltevorrichtungen entfernbar ist. Das Werkzeug kann durch bloßes Ziehen an dem Werkzeug aus der Werkzeughaltevorrichtung entfernt werden. Durch den Entfall der Löse- oder Entriegelungsvorrichtung wird in vorteilhafter Weise die Werkzeughaltevorrichtung, insbesondere deren Höhe oder axiale Abmessung (bezogen auf die Drehachse eines in der Werkzeughaltevorrichtung aufgenommenen Werkzeugs), kleiner und demgemäß verringert sich die Abmessung, insbesondere die Höhe, eines Handstück, insbesondere eines Handstückkopfs, in dem die Werkzeughaltevorrichtung aufgenommen ist.

Die polygonale Drehmomentübertragungsvorrichtung bewirkt eine Drehmomentübertragung auf das Werkzeug durch einen direkten Kontakt zwischen der Drehmomentübertragungsvorrichtung und dem Werkzeug, insbesondere zwischen der mit der Drehmomentübertragungsvorrichtung verbundenen oder diese aufweisenden Werkzeughülse und dem Werkzeug.

Ein weiterer Vorteil der Werkzeughaltevorrichtung besteht darin, dass der Klemmrollen-Haltemechanismus das Werkzeug im Wesentlichen spielfrei, insbesondere axial und radial spielfrei, fixiert, so dass insbesondere auch beim Übertragen hoher Drehmomente ein sicheres, zuverlässiges und präzises Arbeiten möglich ist.

Gemäß einem Ausführungsbeispiel weist die Drehmomentübertragungsvorrichtung eine polygonale Ausnehmung zur Aufnahme des polygonalen Abschnitts des Werkzeugschafts auf. Die polygonale Ausnehmung ist zum Beispiel als dreieckige, viereckige, fünfeckige oder vorzugsweise sechseckige Ausnehmung ausgebildet. Gemäß einem anderen Ausführungsbeispiel ist die Drehmomentübertragungsvorrichtung hülsenförmig ausgebildet, wobei vorzugsweise an der Innenwand der Hülse die polygonale Ausnehmung zur Aufnahme des polygonalen Abschnitts des Werkzeugschafts vorgesehen ist oder die polygonale Ausnehmung durch die Innenwand der Hülse gebildet ist. Vorzugsweise ist die, insbesondere hülsenförmige, Drehmomentübertragungsvorrichtung ausgebildet, einen Formschluss mit dem polygonalen Abschnitt des Werkzeugschafts zu bilden. Der polygonale Abschnitt des Werkzeugschafts ist demgemäß vorzugsweise dreieckig, viereckig, fünfeckig oder bevorzugt sechseckig geformt, insbesondere weist der polygonale Abschnitt des Werkzeugschafts zur Bildung eines Formschlusses die gleiche Form wie die Drehmomentübertragungsvorrichtung auf. Diese genannten Ausführungsbeispiel verbessern in vorteilhafter Weise zusätzlich die Übertragung des Drehmoments auf das Werkzeug.

Gemäß einem Ausführungsbeispiel ist die Drehmomentübertragungsvorrichtung einteilig mit der Werkzeughülse ausgebildet. Vorzugsweise ist die Drehmomentübertragungsvorrichtung an einem Ende der Werkzeughülse angeordnet. Beide Ausführungsbeispiele erleichtern, insbesondere in Kombination, in vorteilhafter Weise die Herstellung der Werkzeughaltevorrichtung. Alternativ ist es natürlich auch möglich, dass die Drehmomentübertragungsvorrichtung und die Werkzeughülse als separate Bauteile hergestellt sind, die drehfest miteinander verbunden sind, zum Beispiel durch Verpressen und / oder Verschweißen. Insbesondere die Anordnung der Drehmomentübertragungsvorrichtung an einem Ende der Werkzeughülse erhöht auch die Qualität der Lagerung des Werkzeugs in der Werkzeughaltevorrichtung, da das Werkzeug, insbesondere der im Querschnitt runde Abschnitt des Werkzeugschafts, über eine große Länge gelagert ist.

Gemäß einem Ausführungsbeispiel ist die lichte Weite der Drehmomentübertragungsvorrichtung, insbesondere die lichte Weite der polygonalen Ausnehmung der Drehmomentübertragungsvorrichtung, zumindest geringfügig größer als der Innendurchmesser zumindest eines Abschnitts der Innenbohrung der Werkzeughülse, der zur Aufnahme zumindest eines Teils des ersten, im Querschnitt runden Abschnitts des Werkzeugschafts ausgebildet ist. Insbesondere wenn zusätzlich die Drehmomentübertragungsvorrichtung an einem Ende der Werkzeughülse angeordnet ist, wird damit eine besonders zuverlässige Lagerung über eine große Länge des Werkzeugs oder des im Querschnitt runden Abschnitts des Werkzeugschafts erreicht.

Gemäß einem Ausführungsbeispiel ist an der Klemmhülse ein Antriebselement vorgesehen, insbesondere ein Zahnrad oder ein druckluftbetriebenes Laufrad, zum in Drehung Versetzen von zumindest Teilen der Werkzeughaltevorrichtung. Gemäß einem anderen Ausführungsbeispiel ist an der Klemmhülse ein erster Lagersitz und an der Werkzeughülse ein zweiter Lagersitz vorgesehen, so dass die Werkzeughaltevorrichtung mittels zweier Lager, insbesondere Wälz- oder Kugellager, drehbar in einem medizinischen, insbesondere dentalen oder chirurgischen, Handstück lagerbar ist. Gemäß einem weiteren Ausführungsbeispiel ragt ein Abschnitt der Werkzeughülse über ein Ende der Klemmhülse hinaus, insbesondere jener Abschnitt der Werkzeughülse, an dem die Drehmomentübertragungsvorrichtung und / oder der zweite Lagersitz vorgesehen ist / sind. Diese drei Ausführungsbeispiele ermöglichen in vorteilhafter Weise eine zusätzliche Verkleinerung der Werkzeughaltevorrichtung und / oder des Handstückkopfs.

Gemäß einem Ausführungsbeispiel weist die Werkzeughaltevorrichtung eine Einspannvorrichtung auf, die ausgebildet ist, das Werkzeug, insbesondere wenn die Werkzeughaltevorrichtung, bevorzugt die Klemmhülse, die zumindest einen Klemmrolle und die Werkzeughülse, nicht in Drehung versetzt sind, in der Werkzeughaltevorrichtung zu halten. Vorzugsweise weist die Einspannvorrichtung zumindest ein Formelement, insbesondere eine Kugel, und ein Federelement auf, wobei das Federelement das Formelement durch eine Querbohrung in der Werkzeughülse in die Innenbohrung der Werkzeughülse vorspannt, so dass ein in der Innenbohrung der Werkzeughülse aufgenommener Abschnitt des Werkzeugschafts durch das zumindest eine Formelement in der Werkzeughülse klemmbar ist. Besonders bevorzugt sind drei Formelemente und drei Querbohrungen vorgesehen. Selbstverständlich kann die Einspannvorrichtung auch andere Bauteile umfassen. Die Einspannvorrichtung hält das Werkzeug insbesondere nach dem Einstecken des Werkzeugs in die Werkzeughaltevorrichtung und vor Beginn des in Drehung Versetzens der Werkzeughaltevorrichtung.

Vorzugsweise ist die Einspannvorrichtung zumindest teilweise in der Klemmhülse aufgenommen. Bevorzugt ist die Einspannvorrichtung an einem Abschnitt der Werkzeughülse vorgesehen, der zur Aufnahme zumindest eines Teils des ersten, im Querschnitt runden Abschnitts des Werkzeugschafts ausgebildet ist. Durch diese beiden Maßnahmen ist die Einspannvorrichtung kompakt in die Werkzeughaltevorrichtung integriert oder integraler Teil der Werkzeughaltevorrichtung, so dass die Werkzeughaltevorrichtung und / oder der Handstückkopf sehr kompakt sind und möglichst geringen Abmessungen haben.

Gemäß einem Ausführungsbeispiel ist die Werkzeughaltevorrichtung in einem medizinischen, insbesondere dentalen oder chirurgischen, Handstück, vorzugsweise zur Verwendung bei implantologischen Behandlungen vorgesehen. Der Begriff Handstück, in welchem die Werkzeughaltevorrichtung aufgenommen ist, umfasst sowohl gerade Handstücke als auch gebogene oder gewinkelte Handstück, oftmals als Winkelstücke bezeichnet. Ein derartiges Winkelstück weist eine Außenhülse auf, die ein Anschlussteil mit einer Kupplungsvorrichtung zum Anschluss an eine Steuer-, Regel- und / oder Versorgungseinheit, ein gebogen oder gewinkelt zum Anschlussteil ausgebildetes Mittelteil und ein an das Mittelteil anschließendes Kopfteil umfasst. Am Kopfteil der Außenhülse ist eine Werkzeugaufnahmeöffnung vorgesehen, an der im Inneren des Kopfteils die Werkzeughaltevorrichtung angeordnet ist. Die Werkzeugaufnahmeöffnung ist seitlich am Kopfteil vorgesehen, so dass die Drehachse eines in der Werkzeughaltevorrichtung aufgenommenen Werkzeugs gewinkelt zu der Längsachse des Mittelteils angeordnet ist.

Insbesondere ist die Werkzeughaltevorrichtung in dem Handstückkopf oder Kopfteil des Handstücks vorgesehen. Vorzugsweise weist das Handstück des Weiteren auf: Eine Außenhülse mit einer Werkzeugaufnahmeöffnung, durch welche ein Werkzeugschaft eines medizinischen, insbesondere dentalen oder chirurgischen, Werkzeugs zumindest teilweise in das Handstück einführbar und daraus entfernbar ist, sowie ein erstes Lager und ein separates, zweites Lager, welche die Werkzeughaltevorrichtung drehbar im Handstück lagern, wobei das zweite Lager näher zur Werkzeugaufnahmeöffnung angeordnet ist als das erste Lager und wobei die Drehmomentübertragungsvorrichtung der Werkzeughaltevorrichtung im Bereich des zweiten Lagers vorgesehen ist, insbesondere im Bereich der Werkzeugaufnahmeöffnung oder in der Werkzeugaufnahmeöffnung. Vorzugsweise ist auch das an der Klemmhülse vorgesehene Antriebselement im Bereich des zweiten Lagers angeordnet. Das erste und das zweite Lager sind insbesondere an dem ersten Lagersitz der Klemmhülse und an dem zweiten Lagersitz der Werkzeughülse vorgesehen.

Aufgrund des Vorsehens der Werkzeughaltevorrichtung in dem Handstück, insbesondere des Klemmrollen-Haltemechanismus und der Einspannvorrichtung, ist es in vorteilhafter Weise nicht notwendig, an der Außenhülse des Handstücks, vorzugsweise auch im Handstück und / oder an der Werkzeughaltevorrichtung, ein Betätigungselement zum Entfernen des Werkzeugs aus der Werkzeughaltevorrichtung oder aus dem Handstück (zum Beispiel einen Druckknopf, einen Drucktaster, eine Entriegelungsvorrichtung oder ähnliches) vorzusehen. Die Werkzeughaltevorrichtung oder das Handstück sind insbesondere derart ausgebildet, dass durch bloßes Ziehen an dem Werkzeug, ohne Betätigung oder Aktivierung eines Löseelements das Werkzeug aus der Werkzeughaltevorrichtung oder aus dem Handstück entfernbar ist, so dass weder an der Werkzeughaltevorrichtung noch an dem Handstück eine Element zum Entfernen des Werkzeugs aus der Werkzeughaltevorrichtung oder aus dem Handstück vorgesehen ist.

Die Erfindung wird nachfolgend anhand bevorzugter Ausführungsbeispiele und Bezug nehmend auf die beigefügten Zeichnungen erläutert:
Figur 1 zeigt eine Schnittdarstellung durch ein erstes Ausführungsbeispiel eines Handstücks mit einer Werkzeughaltevorrichtung, die einen Klemmrollen-Haltemechanismus, eine Drehmomentübertragungsvorrichtung und eine Einspannvorrichtung aufweist.
Figur 2 zeigt eine Außenansicht ein zweites Ausführungsbeispiel eines Handstücks mit einer Werkzeughaltevorrichtung, die einen Klemmrollen-Haltemechanismus, eine Drehmomentübertragungsvorrichtung und eine Einspannvorrichtung aufweist
Figur 3 zeigt eine Schnittdarstellung durch den Klemmrollen-Haltemechanismus entlang der Achse II - II des Handstücks der Figur 2.
Figur 4 zeigt eine Schnittdarstellung durch die Einspannvorrichtung entlang der Achse I - I des Handstücks der Figur 2.

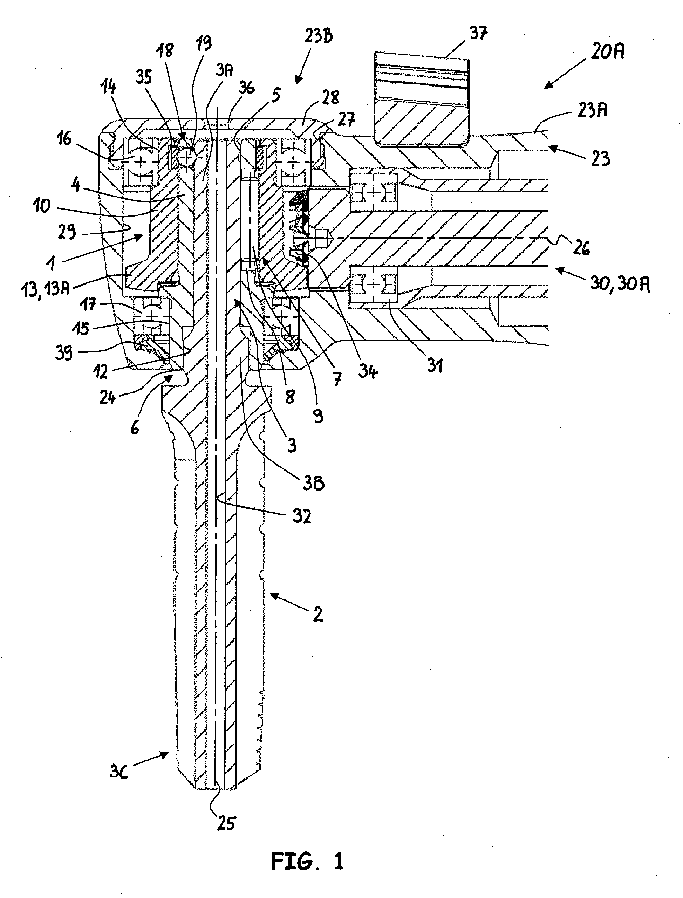

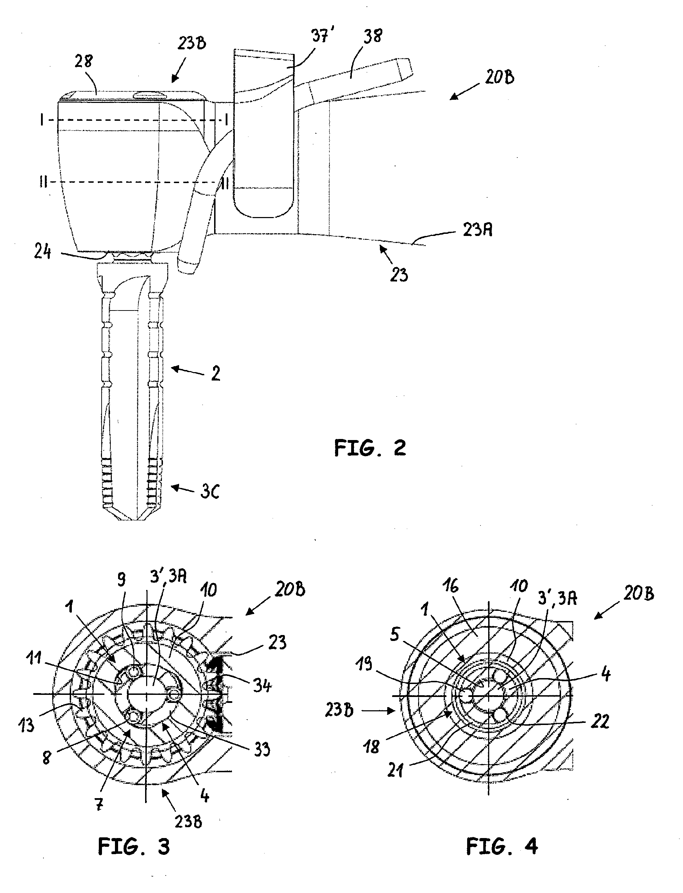

Die Handstücke 20A, 20B der Figuren 1 - 4 sind im Wesentlichen gleich aufgebaut, so dass die folgende Beschreibung auf beide Handstücke 20A, 20B zutrifft. Insbesondere sind die Schnittdarstellung der Figuren 3 und 4 mit Ausnahme der Art des dargestellten Werkzeugschafts 3, 3' in gleicher Weise für beide Handstücke 20A, 20B zutreffend.

Die Handstücke 20A, 20B sind als gebogene oder gewinkelte Handstücke oder Winkelstücke ausgebildet. Die Winkelstücke 20A, 20B weisen eine Außenhülse 23 auf, die ein (nicht dargestelltes) Anschlussteil mit einer Kupplungsvorrichtung zum Anschluss an eine Steuer-, Regel- und / oder Versorgungseinheit, ein gebogenes oder gewinkeltes zum Anschlussteil ausgebildetes Mittelteil 23A und ein an das Mittelteil 23A anschließendes Kopfteil 23B umfasst. Am Kopfteil 23B ist eine Werkzeugaufnahmeöffnung 24 vorgesehen, an der oder anschließend an die im Inneren des Kopfteils 23B eine Werkzeughaltevorrichtung 1 zum lösbaren Halten eines Werkzeugs 2 angeordnet ist. Die Werkzeugaufnahmeöffnung 24 ist seitlich am Kopfteil 23B vorgesehen, so dass die Drehachse 25 eines in der Werkzeughaltevorrichtung 1 aufgenommenen Werkzeugs 2 oder einer zumindest einen Teil des Werkzeugs 2 aufnehmenden Werkzeughülse 4 der Werkzeughaltevorrichtung 1 gewinkelt zu der Längsachse 26 des Mittelteils 23A angeordnet ist.

Zumindest das Mittelteil 23A und das Kopfteil 23B, vorzugsweise auch das Anschlussteil, des Winkelstücks 20A, 20B sind einteilig ausgebildet. Das Kopfteil 23B weist an der der Werkzeugaufnahmeöffnung 24 gegenüber liegenden Seite ein Öffnung 27 auf, die durch eine Verschlusskappe 28 verschließbar ist, so dass im Inneren des Kopfteils 23B eine Kammer 29 gebildet ist. In der Kammer 29 ist eine Werkzeughaltevorrichtung 1 angeordnet, die mittels einer Antriebsvorrichtung 30 in eine Drehbewegung versetzbar ist. Die Antriebsvorrichtung 30 umfasst zum Beispiel eine oder mehrere Wellen 30A, einen Motor, insbesondere einen Elektromotor, oder eine Vorrichtung zur Förderung eines Antriebsfluids. Gemäß der Figur 1 ist die Welle 30A mittels zumindest eines Wälzlagers 31 in dem Mitteilteil 23A der Außenhülse 23 drehbar gelagert.

In der Werkzeughaltevorrichtung 1 ist lösbar ein medizinisches, insbesondere dentales oder chirurgisches, Werkzeug 2 aufgenommen. Das Werkzeug 2 weist einen Werkzeugschaft 3, 3' mit zumindest einem ersten, im Querschnitt runden Abschnitt 3A und einem zweiten, polygonalen Abschnitt 3B sowie einen, vorzugsweise abrasiven, Arbeitsabschnitt 3C auf. Der polygonale Abschnitt 3B ist zur Übernahme des von der Werkzeughaltevorrichtung 1 zur Verfügung gestellten oder übertragenen Drehmoments ausgebildet. Die Querschnitts-Außenbreite des polygonalen Abschnitts 3B ist vorzugsweise zumindest geringfügig größer als der Durchmesser des runden Abschnitts 3A. Das gesamte Werkzeug 2 der Figur 1, d.h. der runde Abschnitt 3A, der polygonale Abschnitt 3B und der Arbeitsabschnitt 3C, weist eine Innenbohrung 32 mit zwei Öffnungen an den beiden Enden des Werkzeugs 2 auf. Im Gegensatz dazu ist das Werkzeug 2 der Figur 2 massiv ausgebildet und hat somit keine Innenbohrung.

Die Werkzeughaltevorrichtung 1 umfasst eine Drehmomentübertragungsvorrichtung 6, die zur Aufnahme des polygonalen Abschnitts 3B des Werkzeugschafts 3, 3' und zur Übertragung eines Drehmoments auf das Werkzeug 2 ausgebildet ist, einen Klemmrollen-Haltemechanismus 7, der zum lösbaren Halten des Werkzeugs 2 und vorzugsweise auch zum Übertragen des Drehmoments auf das Werkzeug 2 ausgebildet ist, und eine Einspannvorrichtung 18, die ausgebildet ist, das Werkzeug 2, insbesondere wenn die Werkzeughaltevorrichtung 1 nicht in Drehung versetzt ist, lösbar, insbesondere axial in Bezug auf die Drehachse 25, in der Werkzeughaltevorrichtung 1 zu halten. Die Werkzeughaltevorrichtung 1 ist mittels eines ersten Lagers 16 und eines zweiten Lagers 17 drehbar in dem Kopfteil 23B gelagert. Die beiden Lager 16, 17 sind axial voneinander beabstandet, vorzugsweise ist das erste Lager 16 an oder nahe an der Verschlusskappe 28 und das zweite Lager 17 im Bereich oder nahe der Werkzeugaufnahmeöffnung 24 angeordnet.

Die Drehmomentübertragungsvorrichtung 6 ist gemäß der Figur 1 als integraler Teil der Werkzeughülse 4 ausgebildet, so dass die Drehmomentübertragungsvorrichtung 6 und die Werkzeughülse 4 ein einziges, hülsenförmiges Bauteil bilden. Die Drehmomentübertragungsvorrichtung 6 ist anschließend an die Werkzeugaufnahmeöffnung 24 in der Kammer 29 und / oder im Bereich des zweiten Lagers 17 angeordnet. Die Drehmomentübertragungsvorrichtung 6 umfasst eine, durch die Innenwand der Drehmomentübertragungsvorrichtung 6 oder der Werkzeughülse 4 gebildete, polygonale, insbesondere hexagonale, Aufnahme oder Ausnehmung 12, in welche der polygonale, vorzugsweise hexagonale, Abschnitt 3B des Werkzeugs 2 einführbar ist. Die Werkzeughaltevorrichtung 1 ist derart ausgebildet, dass, wenn die Werkzeughaltevorrichtung 1 in Drehung versetzt, über die einander kontaktierenden Flächen und / oder Kanten der Ausnehmung 12 und des polygonalen Abschnitt 3B ein Drehmoment von der Werkzeughaltevorrichtung 1 auf das Werkzeug 2 übertragen wird. Ein für das zweite Lager 17 vorgesehener zweiter Lagersitz 15 ist im Bereich oder an der Außenseite der Drehmomentübertragungsvorrichtung 6 vorgesehen.

Im Bereich der Werkzeugaufnahmeöffnung 24 und / oder der Drehmomentübertragungsvorrichtung 6 ist vorzugsweise zumindest ein Dichtelement 39 vorgesehen, zum Beispiel ein Dichtring, der ausgebildet ist, die Werkzeugaufnahmeöffnung 24 abzudichten, um zum Beispiel das Eindringen von Partikeln und Verunreinigungen in das Handstück 20A zu unterbinden. Das Dichtelement 39 ist insbesondere drehfest an dem Kopfteil 23B befestigt und ragt nahe an oder kontaktiert die Werkzeughülse 4.

Der insbesondere in den Figuren 1 und 3 dargestellte Klemmrollen-Haltemechanismus 7 umfasst mehrere, zum Beispiel drei, Klemmrollen 9. Die Klemmrollen 9 sind zylindrisch, vorzugsweise länglich, ausgebildet. Jede Klemmrolle 9 ist in einem eigenen Durchbruch 8 der Werkzeughülse 4 aufgenommen. Zwei Durchbrüche 8 sind durch jeweils einen länglichen Steg 33, der von der Wand der Werkzeughülse 4 gebildet ist, voneinander getrennt.

Der Klemmrollen-Haltemechanismus 7 umfasst des Weiteren eine in eine Drehbewegung versetzbare Klemmhülse 10, welche die Klemmrollen 9 und die Werkzeughülse 4 mit den Durchbrüchen 8 umgibt. An der Innenwand der Klemmhülse 10 ist für jede Klemmrolle 9 eine Vertiefung 11 vorgesehen, die sich im Querschnitt mit verändernder Tiefe in die Innenwand wölbt und in welcher jeweils eine Klemmrolle 9 aufnehmbar ist. Die Klemmhülse 10 ist in Bezug auf die von ihr aufgenommene Werkzeughülse 4 kürzer, d.h. sie weist eine geringere axiale Ausdehnung auf, so dass insbesondere jener Teil der Werkzeughülse 4, an dem der zweite Lagersitz 15 und / oder das zweite Lager 17 und / oder die Drehmomentübertragungsvorrichtung 6 vorgesehen sind, über das Ende der Klemmhülse 10 ragt. Die Klemmhülse 10 und die Werkzeughülse 4 sind gleitend aneinander gelagert, so dass sie relativ zueinander bewegbar sind. An der Klemmhülse 10, insbesondere an einem Ende der Klemmhülse 10, vorzugsweise an dem der Drehmomentübertragungsvorrichtung 6 und / oder dem zweite Lagersitz 15 und / oder dem zweiten Lager 17 nahen Ende der Klemmhülse 10, ist ein Antriebselement 13, zum Beispiel ein Zahnrad 13A, vorgesehen. Das Antriebselement 13 ist mit der Antriebsvorrichtung 30 operativ verbunden, zum Beispiel kämmt das Zahnrad 13A mit einem weiteren Zahnrad 34, das mit der Welle 30A verbunden ist, so dass eine Antriebsbewegung, insbesondere eine Drehbewegung, von der Antriebsvorrichtung 30 auf die Werkzeughaltevorrichtung 1 übertragbar ist.

An der Klemmhülse 10 ist des Weiteren ein erster Lagersitz 14 vorgesehen, an dem das erste Lager 16 gelagert ist. Der erste Lagersitz 14 ist vorzugsweise an einem Ende der Klemmhülse 10 angeordnet, insbesondere an dem von dem Antriebselement 13 entfernten Ende der Klemmhülse 10. Das erste Lager 16 ist vorzugsweise auch an der Verschlusskappe 28 gelagert.

Die Klemmhülse 10 einerseits und die Klemmrollen 9 und die Werkzeughülse 4 andererseits sind derart relativ zueinander drehbar angeordnet, dass durch eine Relativbewegung zwischen der Klemmhülse 10 einerseits und den Klemmrollen 9 und der Werkzeughülse 4 andererseits die Klemmrollen 9 von einer ersten Position mit einer ersten Tiefe in den gewölbten Vertiefungen 11 in eine zweite Position mit einer zweiten Tiefe, die geringer ist als die erste Tiefe, und dadurch in Richtung der Innenbohrung 5 der Werkzeughülse 4 bewegbar sind. Dadurch ragen die Klemmrollen 9 vorzugsweise in die Innenbohrung 5 und kontaktieren oder klemmen einen darin aufgenommenen Werkzeugschaft 3, 3'. Auf diese Weise ist ein in der Werkzeughülse 4 aufgenommener Werkzeugschaft 3, 3' durch den Klemmrollen-Haltemechanismus 7 lösbar in der Werkzeughaltevorrichtung 1 befestigbar.

Die Relativbewegung zwischen der Klemmhülse 10 einerseits und den Klemmrollen 9 und der Werkzeughülse 4 andererseits erfolgt insbesondere beim oder durch die Inbetriebnahme des Handstücks 20A, 20B, d.h. durch die Übertragung der Antriebsbewegung von der Antriebsvorrichtung 30 über das Antriebselement 13, 13A auf die Klemmhülse 10. Die Relativbewegung endet, sobald die Klemmrollen 9 eine Endposition erreicht haben, zum Beispiel die oben genannte zweite Position mit einer zweiten Tiefe innerhalb der Vertiefungen 11 oder eine Position außerhalb der Vertiefungen 11, in welcher sie derart fest zwischen dem Werkzeugschaft 3, 3' des in die Werkzeughülse 4 eingeführten Werkzeugs 2 und der Klemmhülse 10 eingespannt sind, dass die Klemmhülse 10, die Werkzeughülse 4 und das darin aufgenommene Werkzeug 2 gemeinsam rotieren.

Die Einspannvorrichtung 18, die ausgebildet ist, das Werkzeug 2, insbesondere wenn die Werkzeughaltevorrichtung 1 nicht in Drehung versetzt ist, in der Werkzeughaltevorrichtung 1 zu halten, umfasst mehrere, zum Beispiel drei, Formelemente 19, insbesondere Kugeln, und zumindest ein Federelement 21, zum Beispiel einen Federring oder C-Ring. Jedem Formelement 19 ist eine Querbohrung 22 in der Werkzeughülse 4 zugeordnet. Das Federelement 21 spannt die Formelemente 19 durch die Querbohrungen 22 in die Innenbohrung 5 der Werkzeughülse 4 vor, so dass ein in der Innenbohrung 5 der Werkzeughülse 4 aufgenommener Abschnitt des Werkzeugschafts 3, 3' durch die Formelemente 19 in der Werkzeughülse 4 klemmbar ist. Vorzugsweise ist am Werkzeugschaft 3, 3' zumindest eine Aufnahme, zum Beispiel eine Ringnut, für die Formelemente 19 vorgesehen.

Vorzugsweise ist zumindest ein Teil der Einspannvorrichtung 18 zumindest teilweise in der Klemmhülse 10, insbesondere in einer Aufnahme 35 oder einem Rücksprung der Klemmhülse 10, aufgenommen, zum Beispiel zumindest ein Teil des Federelements 21. Vorzugsweise ist die Einspannvorrichtung 18 an einem Abschnitt der Werkzeughülse 4 vorgesehen, der zur Aufnahme zumindest eines Teils des ersten, im Querschnitt runden Abschnitts 3A des Werkzeugschafts 3, 3' ausgebildet ist. Insbesondere ist die Einspannvorrichtung 18 im Bereich des ersten Lagersitzes 14 und / oder an einem Ende der Werkzeughülse 4 und / oder an dem von der Drehmomentübertragungsvorrichtung 6 entfernten Ende der Werkzeughülse 4 und / oder an einem Ende der Klemmhülse 10 angeordnet.

Vorzugsweise sind zumindest Teile der Drehmomentübertragungsvorrichtung 6, des Klemmrollen-Haltemechanismus 7 und der Einspannvorrichtung 18 koaxial angeordnet, insbesondere koaxial zur Drehachse 25.

Wie im Vorstehenden bereits beschrieben, sind die beiden Handstücke 20A, 20B im Wesentlichen baugleich, insbesondere ist die Werkzeughaltevorrichtung 1 in beiden Handstücken 20A, 20B ident. Der wesentliche Unterschied zwischen den beiden Handstücken 20A, 20B besteht in der Kühlmittelversorgung: Das Handstück 20A der Figur 1 weist eine interne Kühlmittelversorgung auf, bei welcher ein mit einer Kühlmittelquelle verbundener Schlauch durch eine Öffnung 36 in der Verschlusskappe 28 ein Kühlmittel in die Innenbohrung 32 des Werkzeugs 2 abgibt. Das Kühlmittel gelangt durch die endseitige Öffnung der Innenbohrung 32 am Arbeitsabschnitt 3C auf die Behandlungsstelle. Der Kühlmittelschlauch kann mittels einer Befestigungsvorrichtung 37, zum Beispiel einer Klammer, lösbar am Handstück 20A befestigt werden.

Das Handstück 20B der Figur 2 umfasst eine externe Kühlmittelversorgung, bei welcher ein mit einer Kühlmittelquelle verbundener Schlauch und / oder eine Rohr 38 an der Außenseite des Handstücks 20B geführt ist / sind. Das Ende des Rohrs 38 weist in Richtung des Werkzeugs 2, insbesondere des Arbeitsabschnitts 3C, so dass das Kühlmittel auf das Werkzeug 2 und / oder die Behandlungsstelle abgebbar ist. Der Schlauch und / oder das Rohr 38 sind mittels einer Befestigungsvorrichtung 37', zum Beispiel einer Klammer, am Handstück 20B lösbar befestigbar. Alternativ ist selbstverständlich auch ein Handstück mit einer externen und einer internen Kühlmittelversorgung und einer Werkzeughaltevorrichtung 1 vorstellbar.

Die Erfindung ist nicht auf die beschriebenen Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Werkzeughaltevorrichtung (1) zum Halten eines medizinischen, insbesondere dentalen oder chirurgischen, Werkzeugs (2) mit einem Werkzeugschaft (3, 3'), der zumindest einen ersten, im Querschnitt runden Abschnitt (3A) und einen zweiten, polygonalen Abschnitt (3B) aufweist, wobei die Werkzeughaltevorrichtung (1) umfasst: Eine in eine Drehbewegung versetzbare Werkzeughülse (4), deren Innenbohrung (5) zur Aufnahme zumindest eines Teils des ersten, im Querschnitt runden Abschnitts (3A) des Werkzeugschafts (3, 3') ausgebildet ist, wobei die Werkzeughaltevorrichtung weiterhin umfasst einen Klemmrollen-Haltemechanismus (7), der aufweist: Zumindest eine, in einem Durchbruch (8) der Werkzeughülse (4) aufgenommene Klemmrolle (9) und eine in eine Drehbewegung versetzbare Klemmhülse (10), welche die zumindest eine Klemmrolle (9) und die Werkzeughülse (4) umgibt, wobei an der Innenwand der Klemmhülse (10) eine im Querschnitt mit sich verändernder Tiefe in die Innenwand gewölbte Vertiefung (11) vorgesehen ist, in welcher die Klemmrolle (9) aufnehmbar ist, und wobei die Klemmhülse (10) einerseits und die zumindest eine Klemmrolle (9) und die Werkzeughülse (4) andererseits derart relativ zueinander drehbar angeordnet sind, dass **durch** eine Relativbewegung zwischen der Klemmhülse (10) einerseits und der zumindest einen Klemmrolle (9) und der Werkzeughülse (4) andererseits die zumindest eine Klemmrolle (9) von einer ersten Position mit einer ersten Tiefe in der gewölbten Vertiefung (11) der Innenwand der Klemmhülse (10) in eine zweite Position mit einer zweiten Tiefe, die geringer ist als die erste Tiefe, und **dadurch** in Richtung der Innenbohrung (5) der Werkzeughülse (4) bewegbar ist, so dass ein in der Werkzeughülse (4) aufgenommener Werkzeugschaft (3, 3') **durch** den Klemmrollen-Haltemechanismus (7) lösbar in der Werkzeughaltevorrichtung (1) befestigbar ist, **dadurch gekennzeichnet, dass** die Werkzeughaltevorrichtung weiterhin umfasst eine mit der Werkzeughülse (4) drehfest verbundene Drehmomentübertragungsvorrichtung (6), die zur Aufnahme des zweiten, polygonalen Abschnitts (3B) des Werkzeugschafts (3, 3') und zur Übertragung eines Drehmoments auf das Werkzeug (2) ausgebildet ist.

2. Werkzeughaltevorrichtung (1) nach Anspruch 1, **dadurch gekennzeichnet, dass** die Drehmomentübertragungsvorrichtung (6) eine polygonale Ausnehmung (12) zur Aufnahme des polygonalen Abschnitts (3B) des Werkzeugschafts (3, 3') aufweist.

3. Werkzeughaltevorrichtung (1) nach Anspruch 1 oder 2, **gekennzeichnet durch** eine hülsenförmige Drehmomentübertragungsvorrichtung (6), an deren Innenwand vorzugsweise die polygonale Ausnehmung (12) zur Aufnahme des polygonalen Abschnitts (3B) des Werkzeugschafts (3, 3') vorgesehen ist.

4. Werkzeughaltevorrichtung (1) nach einem der vorstehenden Ansprüche, **dadurch gekennzeichnet, dass**
die Drehmomentübertragungsvorrichtung (6) einteilig mit der Werkzeughülse (4) ausgebildet ist.

5. Werkzeughaltevorrichtung (1) nach einem der vorstehenden Ansprüche, **dadurch gekennzeichnet, dass**
die lichte Weite der Drehmomentübertragungsvorrichtung (6), insbesondere die lichte Weite der polygonalen Ausnehmung der Drehmomentübertragungsvorrichtung (6), zumindest geringfügig größer ist als der Innendurchmesser zumindest eines Abschnitts der Innenbohrung (5) der Werkzeughülse (4), der zur Aufnahme zumindest eines Teils des ersten, im Querschnitt runden Abschnitts (3A) des Werkzeugschafts (3, 3') ausgebildet ist.

6. Werkzeughaltevorrichtung (1) nach einem der vorstehenden Ansprüche, **gekennzeichnet durch**
ein an der Klemmhülse (10) vorgesehenes Antriebselement (13), insbesondere ein Zahnrad (13A), zum in Drehung Versetzen von zumindest Teilen der Werkzeughaltevorrichtung (1).

7. Werkzeughaltevorrichtung (1) nach einem der vorstehenden Ansprüche, **dadurch gekennzeichnet, dass**
an der Klemmhülse (10) ein erster Lagersitz (14) und an der Werkzeughülse (4) ein zweiter Lagersitz (15) vorgesehen ist, so dass die Werkzeughaltevorrichtung (1) mittels zweier Lager (16, 17) drehbar in einem medizinischen, insbesondere dentalen oder chirurgischen, Handstück (20A, 20B) lagerbar ist.

8. Werkzeughaltevorrichtung (1) nach einem der vorstehenden Ansprüche, **dadurch gekennzeichnet, dass**
ein Abschnitt der Werkzeughülse (4) über ein Ende der Klemmhülse (10) hinaus ragt, insbesondere jener Abschnitt der Werkzeughülse (4), an dem die Drehmomentübertragungsvorrichtung (6) und / oder der zweite Lagersitz (15) vorgesehen ist / sind.

9. Werkzeughaltevorrichtung (1) nach einem der vorstehenden Ansprüche, **dadurch gekennzeichnet, dass**
die Werkzeughaltevorrichtung (1) eine Einspannvorrichtung (18) aufweist, die ausgebildet ist, das Werkzeug (2), insbesondere wenn die Werkzeughaltevorrichtung (1) nicht in Drehung versetzt ist, in der Werkzeughaltevorrichtung (1) zu halten.

10. Werkzeughaltevorrichtung (1) nach Anspruch 9, **dadurch gekennzeichnet, dass** die Einspannvorrichtung (18) zumindest ein Formelement (19), insbesondere eine Kugel, und ein Federelement (21) aufweist, wobei das Federelement (21) das Formelement (19) durch eine Querbohrung (22) in der Werkzeughülse (4) in die Innenbohrung (5) der Werkzeughülse (4) vorspannt, so dass ein in der Innenbohrung (5) der Werkzeughülse (4) aufgenommener Abschnitt des Werkzeugschafts (3, 3') durch das zumindest eine Formelement (19) in der Werkzeughülse (4) klemmbar ist.

11. Werkzeughaltevorrichtung (1) nach Anspruch 9 oder 10, **dadurch gekennzeichnet, dass**
die Einspannvorrichtung (18) zumindest teilweise in der Klemmhülse (10) aufgenommen ist.

12. Werkzeughaltevorrichtung **(1)** nach Anspruch 9, 10 oder 11, **dadurch gekennzeichnet, dass**
die Einspannvorrichtung (18) an einem Abschnitt der Werkzeughülse (4) vorgesehen ist, der zur Aufnahme zumindest eines Teils des ersten, im Querschnitt runden Abschnitts (3A) des Werkzeugschafts (3, 3') ausgebildet ist.

13. Medizinisches, insbesondere dentales oder chirurgisches, Handstück (20A, 20B), vorzugsweise zur Verwendung bei implantologischen Behandlungen, **gekennzeichnet durch**
eine Werkzeughaltevorrichtung (1) nach einem der vorstehenden Ansprüche.

14. Medizinisches, insbesondere dentales oder chirurgisches, Handstück (20A, 20B) nach Anspruch 13, **dadurch gekennzeichnet, dass**
das Handstück (20A, 20B) des Weiteren aufweist: Eine Außenhülse (23) mit einer Werkzeugaufnahmeöffnung (24), durch welche ein Werkzeugschaft (3, 3') eines medizinischen, insbesondere dentalen oder chirurgischen, Werkzeugs (2) zumindest teilweise in das Handstück (20A, 20B) einführbar und daraus entfernbar ist, sowie ein erstes Lager (16) und ein separates, zweites Lager (17), welche die Werkzeughaltevorrichtung (1) drehbar in dem Handstück (20A, 20B) lagern, wobei das zweite Lager (17) näher zur Werkzeugaufnahmeöffnung (24) angeordnet ist als das erste Lager (16) und wobei die Drehmomentübertragungsvorrichtung (6) der Werkzeughaltevorrichtung (1) im Bereich des zweiten Lagers (17) vorgesehen ist, insbesondere im Bereich der Werkzeugaufnahmeöffnung (24) oder in der Werkzeugaufnahmeöffnung (24).

15. Medizinisches, insbesondere dentales oder chirurgisches, Handstück (20A, 20B) nach Anspruch 13 oder 14, **dadurch gekennzeichnet, dass**
an der Außenhülse (23) des Handstücks (20A, 20B), vorzugsweise auch im Handstück (20A, 20B) und / oder an der Werkzeughaltevorrichtung (1), kein Betätigungselement zum Entfernen des Werkzeugs (2) aus der Werkzeughaltevorrichtung (1) oder aus dem Handstück (20A, 20B) vorgesehen ist.

## Claims

1. A tool-holding device (1) for holding a medical, in particular a dental or surgical tool (2) having a tool shaft (3, 3') which has at least a first section (3A) having a round cross section and a second section (3B) that is polygonal, wherein the tool-holding device (1) comprises: a tool sleeve (4), which can be induced to a rotational movement, its inside bore (5) being designed to accommodate at least a part of the first section (3A) of the tool shaft (3, 3') having a round cross section, wherein the tool-holding device (1) additionally comprises a clamp-roller-holding mechanism (7), which has: at least one clamp roller (9) accommodated in an opening (8) in the tool sleeve (4) and a clamp sleeve (10) that can be induced to a rotational movement and surrounds the at least one clamp roller (9) and the tool sleeve (4), wherein a depression (11) which curves into the inside wall with a varying depth in cross section is provided on the inside wall of the clamp sleeve (10), so that the clamp roller (9) can be accommodated in this depression (11), and wherein the clamp sleeve (10), on the one hand, and the at least one clamp roller (9) and the tool sleeve (4), on the other hand, are arranged to be rotatable relative to one another, such that by a relative movement between the clamp sleeve (10), on the one hand, and the at least one clamp roller (9) on the tool sleeve (4), on the other hand, the at least one clamp roller (9) can be moved from a first position with a first depth in the curved depression (11) in the inside wall of the clamp sleeve (10) into a second position at a second depth, which is less than the first depth and can thereby be moved in the direction of the internal bore (5) in the tool sleeve (4), so that a tool shaft (3, 3') accommodated in the tool sleeve (4) can be secured releasably in the tool-holding device (1) by the clamp-roller-holding mechanism (7), **characterized in that**
the tool-holding device (1) additionally comprises a torque-transmitting device (6), which is connected to the tool sleeve (4) in a rotationally fixed manner and is designed to receive the second polygonal section (3B) of the tool shaft (3, 3') and to transmit a torque to the tool (2).

2. The tool-holding device (1) according to Claim 1, **characterized in that**
the torque-transmitting device (6) comprises a polygonal recess (12) to receive the polygonal section (3B) of the tool shaft (3, 3').

3. The tool-holding device (1) according to Claim 1 or 2, **characterized by**
a sleeve-shaped torque-transmitting device (6) on whose inside wall the polygonal recess (12) for receiving the polygonal section (3B) of the tool shaft (3, 3') is preferably provided.

4. The tool-holding device (1) according to any one of the preceding claims, **characterized in that**
the torque-transmitting device (6) is designed in one piece with the tool sleeve (4).

5. The tool-holding device (1) according to any one of the preceding claims, **characterized in that**
the clear width of the torque-transmitting device (6), in particular the clear width of the polygonal recess of the torque-transmitting device (6), is at least slightly larger than the clear width of at least a section of the inside bore (5) of the tool sleeve (4), which is formed to receive at least a part of the first section (3A) that has a round cross section of the tool shaft (3, 3').

6. The tool-holding device (1) according to any one of the preceding claims, **characterized by**
a drive element (13), which is provided on the clamp sleeve (10), in particular a gearwheel (13A), for inducing rotation of at least parts of the tool-holding device (1).

7. The tool-holding device (1) according to any one of the preceding claims, **characterized in that**
a first bearing seat (14) is provided on the clamp sleeve (10) and a second bearing seat (15) is provided on the tool sleeve (4), so that the tool-holding device (1) can be supported rotatably by means of two bearings (16, 17) in a medical, in particular a dental or surgical handpiece (20A, 20B).

8. The tool-holding device (1) according to any one of the preceding claims, **characterized in that**
a section of the tool sleeve (4) protrudes beyond an end of the clamp sleeve (10), in particular that section of the tool sleeve (4) on which the torque-transmitting device (6) and/or the second bearing seat (15) is/are provided.

9. The tool-holding device (1) according to any one of the preceding claims, **characterized in that**
the tool-holding device (1) comprises a clamping device (18), which is designed to hold the tool (2), in particular when the tool-holding device (1) is not being induced to rotate, in the tool-holding device (1).

10. The tool-holding device (1) according to Claim 9, **characterized in that**
the clamping device (18) comprises at least one form element (19), in particular a sphere and a spring element (21), wherein the spring element (21) prestresses the form element (19) through a transverse bore (22) in the tool sleeve (4) into the inside bore (5) of the tool sleeve (4), so that a section of the tool shaft (3, 3') accommodated in the inside bore (5) of the tool sleeve (4) can be clamped by the at least one form element (19) in the tool sleeve (4).

11. The tool-holding device (1) according to Claim 9 or 10, **characterized in that**
the clamping device (18) is accommodated at least partially in the clamp sleeve (10).

12. The tool-holding device (1) according to Claims 9, 10 or 11, **characterized in that**
the clamping device (18) is provided on a section of the tool sleeve (4), which is designed for accommodating at least a part of the first section (3A) of the tool shaft (3, 3') having a round cross-section.

13. A medical, in particular dental or surgical handpiece (20A, 20B), preferably for use in implantological treatments, **characterized by**
a tool-holding device (1) according to any one of the preceding claims.

14. The medical, in particular dental or surgical handpiece (20A, 20B) according to Claim 13, **characterized in that**
the handpiece (20A, 20B) additionally comprises: an outer sleeve (23) with a tool receptacle opening (24), through which a tool shaft (3, 3') of a medical, in particular dental or surgical tool (2) can be inserted at least partially into the handpiece (20A, 20B) and can be removed from it as well as a first bearing (16) and a separate second bearing (17) which support the tool-holding device (1) rotatably in the handpiece (20A, 20B), wherein the second bearing (17) is arranged closer to the tool receptacle device (24) than the first bearing (16) and wherein the torque-transmitting device (6) of the tool-holding device (1) is provided in the area of the second bearing (17), in particular in the area of the tool receptacle opening (24) or in the tool receptacle opening (24).

15. The medical, in particular dental or surgical handpiece (20A, 20B) according to Claim 13 or 14, **characterized in that**
no actuating element for removing the tool (2) from the tool-holding device (1) or from the handpiece (20A, 20B) is provided on the outer sleeve (23) of the handpiece (20A, 20B), preferably also not in the handpiece (20A, 20B) and/or on the tool-holding device (1).

## Revendications

1. Dispositif porte-outil (1) permettant de tenir un outil médical (2), en particulier dentaire ou chirurgical, comportant une tige d'outil (3 , 3') qui présente au moins un premier segment de section transversale ronde (3A) et un second segment polygonal (3B), ce dispositif porte-outil (1) comprenant: un fourreau à outil (4) pouvant être mis en mouvement de rotation, dont l'alésage intérieur (5) est conçu pour recevoir au moins une partie du premier segment de section transversale ronde (3A) de la tige d'outil (3 , 3'), le dispositif porte-outil (1) comprenant en outre un mécanisme de retenue de rouleau de serrage (7) qui présente: au moins un rouleau de serrage (9) reçu dans une percée (8) du fourreau à outil (4) et un fourreau de serrage (10) pouvant être mis en mouvement de rotation et entourant l'au moins un rouleau de serrage (9) et le fourreau à outil (4), étant prévu, sur la paroi intérieure du fourreau de serrage (10), un renfoncement incurvé (11) dans la paroi intérieure et de profondeur variable dans sa section transversale, dans lequel peut être reçu le rouleau de serrage (9), et le fourreau de serrage (10) d'une part et l'au moins un rouleau de serrage (9) et le fourreau à outil (4) étant d'autre part disposés de manière à pouvoir tourner l'un par rapport à l'autre de manière à ce que, par un mouvement relatif entre le fourreau à outil (4) d'une part et l'au moins un rouleau de serrage (9) et le fourreau à outil (4) d'autre part, l'au moins un rouleau de serrage (9) puisse se déplacer depuis une première position à une première profondeur dans le renfoncement incurvé (11) de la paroi intérieure du fourreau de serrage (10) jusqu'à une seconde position à une seconde profondeur qui est inférieure à la première profondeur et ainsi en direction de l'alésage intérieur (5) du fourreau à outil (4) de manière à ce qu'une tige d'outil (3, 3') reçue dans le fourreau à outil (4) puisse être fixée de manière dissociable par le mécanisme de retenue de rouleau de serrage (7) dans le dispositif porte-outil (1), **caractérisé en ce que**
le dispositif porte-outil (1) comprend en outre un dispositif de transmission de couple de rotation (6) raccordé de manière fixe en rotation au fourreau à outil (4) et qui est conçu pour recevoir le second segment polygonal (3B) de la tige d'outil (3, 3') et pour transmettre un couple de rotation à l'outil (2).

2. Dispositif porte-outil (1) selon la revendication 1, **caractérisé en ce que**
le dispositif de transmission de couple de rotation (6) présente un évidement polygonal (12) destiné à recevoir le segment polygonal (3B) de la tige d'outil (3, 3').

3. Dispositif porte-outil (1) selon la revendication 1 ou 2, **caractérisé par**
un dispositif de transmission de couple de rotation (6) de forme tubulaire sur la paroi duquel est prévu de préférence l'évidement polygonal (12) destiné à recevoir le segment polygonal (3B) de la tige d'outil (3, 3').

4. Dispositif porte-outil (1) selon une des revendications précédentes, **caractérisé en ce que**
le dispositif de transmission de couple de rotation (6) forme un bloc avec le fourreau à outil (4).

5. Dispositif porte-outil (1) selon une des revendications précédentes, **caractérisé en ce que**
le diamètre intérieur du dispositif de transmission de couple de rotation (6), en particulier le diamètre intérieur de l'évidement polygonal (12) du dispositif de transmission de couple de rotation (6), est au moins légèrement supérieur au diamètre intérieur d'au moins un segment de l'alésage intérieur (5) du fourreau à outil (4) qui est conçu pour recevoir au moins une partie du premier segment de section transversale ronde (3A) de la tige d'outil (3, 3').

6. Dispositif porte-outil (1) selon une des revendications précédentes, **caractérisé par**
un élément moteur (13), en particulier une roue crantée (13A), prévu sur le fourreau de serrage (10) pour déplacer en rotation au moins des parties du dispositif porte-outil (1).

7. Dispositif porte-outil (1) selon une des revendications précédentes, **caractérisé en ce**
**qu'**il est prévu au niveau du fourreau de serrage (10) une première chaise de palier (14) et du fourreau à outil (4) une seconde chaise de palier (15), de sorte que le dispositif porte-outil (1) peut s'appuyer en rotation dans une pièce manuelle (20A, 20B) médicale, en particulier dentaire ou chirurgicale au moyen de deux paliers (16, 17).

8. Dispositif porte-outil (1) selon une des revendications précédentes, **caractérisé en ce**
**qu'**un segment du fourreau à outil (4) dépasse d'une extrémité du fourreau de serrage (10), en particulier le segment du fourreau à outil (4) sur lequel est ou sont prévus le dispositif de transmission de couple de rotation (6) et/ou la seconde chaise de palier (15).

9. Dispositif porte-outil (1) selon une des revendications précédentes, **caractérisé en ce que**
le dispositif porte-outil (1) présente un dispositif de serrage (18) qui est conçu pour maintenir l'outil (2) dans le dispositif porte-outil (1), en particulier lorsque le dispositif porte-outil (1) n'est pas mis en rotation.

10. Dispositif porte-outil (1) selon la revendication 9, **caractérisé en ce que**
le dispositif de serrage (18) présente au moins un élément moulé (19), en particulier une bille, et un élément à ressort (21), l'élément à ressort (21) pré-serrant l'élément moulé (19) dans un alésage transversal (22) du fourreau à outil (4) dans l'alésage intérieur (5) du fourreau à outil (4) de manière à ce qu'un segment reçu dans l'alésage intérieur (5) du fourreau à outil (4) puisse être bloqué par l'au moins un élément moulé (19) dans le fourreau à outil (4).

11. Dispositif porte-outil (1) selon la revendication 9 ou 10, **caractérisé en ce que**
le dispositif de serrage (18) est reçu au moins partiellement dans le fourreau de serrage (10).

12. Dispositif porte-outil (1) selon la revendication 9, 10 ou 11, **caractérisé en ce que**
le dispositif de serrage (18) est prévu au niveau d'un segment du fourreau à outil (4) qui est destiné à recevoir au moins une partie du premier segment de section transversale ronde (3A) de la tige d'outil (3, 3').

13. Pièce manuelle médicale (20A, 20B), en particulier dentaire ou chirurgicale, destinée de préférence à une utilisation dans des traitements implantologiques, **caractérisée par**
un dispositif porte-outil (1) selon une des revendications précédentes.

14. Pièce manuelle médicale (20A, 20B), en particulier dentaire ou chirurgicale, selon la revendication 13, **caractérisée en ce que**
la pièce manuelle (20A, 20B) présente en outre: un fourreau extérieur (23) doté d'un orifice récepteur d'outil (24) dans lequel une tige d'outil (3, 3') d'un outil (2) médical, en particulier dentaire ou chirurgical, peut être introduite au moins partiellement dans la pièce manuelle (20A, 20B) et en être retirée, de même qu'un premier palier (16) et un second palier séparé (17) sur lesquels le dispositif porte-outil (1) s'appuie en rotation dans la pièce manuelle (20A, 20B), le second palier (17) étant disposé plus près de l'orifice récepteur d'outil (24) que le premier palier (16) et le dispositif de transmission de couple de rotation (6) du dispositif porte-outil (1) étant prévu au niveau du second palier (17), en particulier au niveau de l'orifice récepteur d'outil (24) ou dans l'orifice récepteur d'outil (24).

15. Pièce manuelle médicale (20A, 20B), en particulier dentaire ou chirurgicale, selon la revendication 13 ou 14, **caractérisée en ce que**,
au niveau du fourreau extérieur (23) de la pièce manuelle (20A, 20B), de préférence aussi dans la pièce manuelle (20A, 20B) et/ou au niveau du dispositif porte-outil (1), aucun élément d'actionnement permettant le retrait de l'outil du dispositif porte-outil (1) ou de la pièce manuelle (20A, 20B) n'est prévu.
